# EUROPEAN PATENT APPLICATION

(11) **EP 1 941 902 A1**
(43) Date of publication of application: **09.07.2008**
(21) Application number: 07100010.3
(22) Date of filing: 02.01.2007
(51) Int. Cl.: A61K 38/31, A61P 25/06

(54) **Use of Somatostatin analogs in cluster headache**

(71) Applicant: Novartis AG, 4002 Basel (CH)
(72) Inventor: The designation of the inventor has not yet been filed
(74) Representative: Graff, Alan

(57) **Abstract**

The present invention relates to the use of a Somatostatin (SRIF) analog which has a high binding affinity to human SSTR1,2,3,5, or a pharmaceutically acceptable salt thereof for the preparation of a pharmaceutical composition for the treatment of cluster headache.

## Description

The present invention relates to a new use of Somatostatin (SRIF) peptidomimetics (also referred to as Somatostatin- or SRIF-analogs).

Somatostatin is a tetradecapeptide having the structure

H-Ala-Gly-Cys-Lys-Asn-Phe-Phe-Trp-Lys-Thr-Phe-Thr-Ser-Cys-OH
1 2 3 4 5 6 7 8 9 10 11 12 13 14

The somatostatin class is a known class of small peptides comprising the naturally occurring somatostatin-14 and analogues having somatostatin related activity, e.g. as disclosed by A.S. Dutta in Small Peptides, Vol.19, Elsevier (1993). By "somatostatin analog" as used herein is meant any straight-chain or cyclic polypeptide having a structure based on that of the naturally occurring somatostatin-14 wherein one or more amino acid units have been omitted and/or replaced by one or more other amino radical(s) and/or wherein one or more functional groups have been replaced by one or more other functional groups and/or one or more groups have been replaced by one or several other isosteric groups. In general, the term covers all modified derivatives of the native somatostatin-14 which exhibit a somatostatin related activity, e.g. they bind to at least one of the five somatostatin receptor (SSTR), preferably in the nMolar range.

Natural somatostatin binds and activates all 5 somatostatin receptors (SSTR1-5) with nmol efficacy and thus causes its multiple physiological effects.

Synthetically available somatostatin analogs differ in their binding affinity to the different somatostatin receptor subtypes and often bind selectively to one or few subtypes with significantly higher affinity.

Somatostatin analogs of particular interest according to the present invention have a high binding affinity to human SSTR1,2,3,5 and have been described e.g. in WO 97/01579, the contents of which being incorporated herein by reference. Said somatostatin analogs comprise the amino acid sequence of formula I

-(D/L)Trp-Lys-X₁ -X₂ - I

wherein X₁ is a radical of formula (a) or (b) 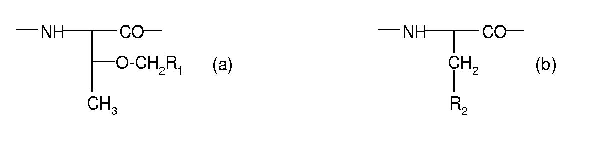 wherein R₁ is optionally substituted phenyl, wherein the substituent may be halogen, methyl, ethyl, methoxy or ethoxy,
R₂ is -Z₁-CH₂-R₁, -CH₂-CO-O-CH₂-R₁, 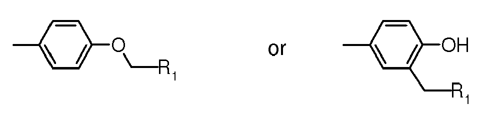 wherein Z₁ is O or S, and
X₂ is an α-amino acid having an aromatic residue on the C_{α} side chain, or an amino acid unit selected from Dab, Dpr, Dpm, His,(Bzl)HyPro, thienyl-Ala, cyclohexyl-Ala and t-butyl-Ala, the residue Lys of said sequence corresponding to the residue Lys⁹ of the native somatostatin-14.

Somatostatin analogs of particular interest which have a high binding affinity to human SSTR1,2,3,5 have also been described e.g. in WO02/10192, the contents of which being incorporated herein by reference. Said somatostatin analogs comprise the compound of formula 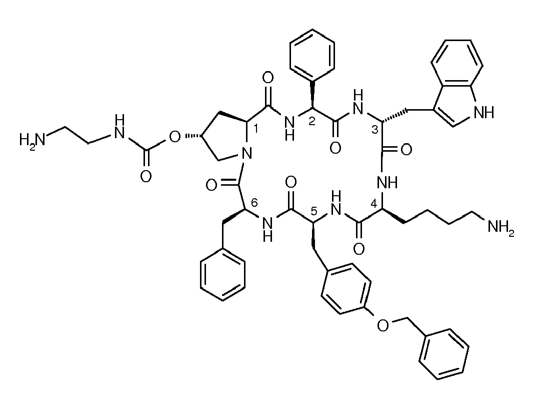 also called cyclo[{4-(NH₂-C₂H₄-NH-CO-O-)Pro}-Phg-DTrp-Lys-Tyr(4-Bzl)-Phe] or pasireotide, as well as diastereoisomers and mixtures thereof, in free form, in salt or complex form or in protected form. Phg means -HN-CH(C₆H₅)-CO- and Bzl means benzyl.

Especially preferred according to the present invention is the use of pasireotide or a pharmaceutically acceptable salt thereof.

Cluster headache (CH) is the most severe form of primary neurovascular headache. It is characterized by frequent episodes of excruciating pain lasting 15 to 180 minutes. Controlled evidence exists to treat acute attacks of CH with oxygen inhalation, intranasal and injectable sumatriptan, high-dose oral zolmitriptan, and intranasal dihydroergotamine. An unequivocally nonvasoconstrictor treatment for acute CH is not available (M. S. Matharu et al., Ann Neurol 2004; 56; 488-494).

Inhalation of oxygen is effective in some patients and safe but is impractical to use.

Injectable and intranasal sumatriptan are highly effective with a rapid onset of action, are portable, and have no tachyphylaxis even with frequent use in prolonged cluster bouts. However, the drawbacks of sumatriptan include the need for an injection with the subcutaneous formulation, the limitation of the number of daily doses that can be administered, the incidence of adverse effects especially with the subcutaneous formulation, and the considerable cost of each dose.

Oral zolmitriptan has been demonstrated to be of only modest efficacy in acute episodic cluster headache at relatively high dose when compared with its use in migraine, thereby rendering it of limited utility in clinical practice.

Intranasal dihydroergotamine has been reported to be better than placebo, but the time to onset of response was not defined and the study used pre-International Headache Society (IHS) diagnostic criteria. In addition, ergots and triptans are contraindicated in patients with vascular disease. Caution must be exercised in patients with CH, because the disorder predominates in middle-aged men, who often have risk factors for cardiovascular disease, particularly smoking.

Two small randomized, double-blind trials suggested efficacy of somatostatin in cluster headache. The problem with studying native somatostatin as a potential abortive agent for CH is that its short half-life of several minutes necessitates an intravenous infusion.

Octreotide, a octapeptide somatostatin analog with a longer half-life of approximately 1.5 hours, can be given subcutaneously, and has been studied as an abortive agent for the acute treatment of CH (M. S. Matharu et al., Ann Neurol. 2004; 56; 488-494).

However, octreotide is a relatively large lipophilic molecule (consisting of 8 amino acids) which preferentially binds to SSTR2 and, only to a lesser extent, to SSTR3 and SSTR5. Therefore, the efficacy of the octreotide treatment of CH is only limited.

Given the limitations of the available agents, some patients do not have an acceptable abortive treatment option. There is therefore a compelling need to develop new pharmacological approaches to treat CH, particularly, if possible, approaches without vascular effects, to effectively and safely treat these patients

Surprisingly, it has been found that the compounds according to the present invention, which have a high binding affinity to several SSTR, especially SSTR1,2,3,5, e.g. pasireotide, have a beneficial relief effect on cluster headache.

This is especially beneficial due to the known pharmacokinetic differences of octreotide (t_{1/2}= 90 min) and pasireotide (t_{1/2}= 11 h) (Schmid and Silva, 2005, J. Endocrine. Invest. 28:28-35), the extended half-time of pasireotide being most valuable for patients.

In addition, pasireotide is a smaller molecule than octreotide (consisting of 8 vs. 6 amino acids) and is less lipophilic than octreotide (partition coefficient log p = 2.7 vs. 5.5). Both factors contribute to a better cell permeability of pasireotide which thus could reach pain sensitive structures within the central nervous system by crossing the blood brain barrier more easily than octreotide.

Although the causes underlying CH attacks are not fully known, it is likely, that nitric oxide can contribute and even induce CH attacks (Goadsby; Curr Opin Neurol 18:283-288; 2005). It has been shown that activation of SSTR5 by somatostatin analogues does inhibit the synthesis of nitric oxide (Cordelier et al. JBC 281:19156-19171). Since pasireotide has a 39-fold higher affinity and a 153-fold higher functional activity than octreotide on the SSTR5 subtype (Schmid et al., Neuroendocrinol. 2004;80:47-50) this further indicates the superiority of pasireotide compared to octreotide in this respect

Compounds which have a very high binding affinity at SSTR5, in addition to their high binding affinity at SSTR1, 2 and 3, like pasireotide, have been shown to have a stronger inhibitory effect on the secretion of several hormones (e.g. GH, GH dependent and GH independent IGF-1 secretion, ACTH, cortisol resp. corticosterone) with less signs of tachyphylaxis compared to compounds which are predominately targeting SSTR2 (and to a lesser extent SSTR5), like octreotide. This offers the possibility that compounds like pasireotide are also active in patients which express mainly SSTR5 and less or no SSTR2.

The increased potency of pasireotide vs. octreotide has been demonstrated in octreotide resistant acromegaly patients and in patients with primary Cushing's disease, a disease in which octreotide was not effective.

In a further aspect, the present invention relates to the use of a Somatostatin (SRIF) analog which has a high binding affinity to human SSTR1,2,3,5, or a pharmaceutically acceptable salt thereof for the preparation of a pharmaceutical composition for the treatment of cluster headache.

The term "SRIF-analog with a high binding affinity to human SSTR1,2,3,5" as used herein (also referred to as COMPOUND OF THE INVENTION) refers to compounds which have a high binding affinity to SSTR1, SSTR2, SSTR3 and SSTR5, preferentially an lC50 < 10 nmol/I at SSTR1 and SSTR2 and an IC50 < 3 nmol/I at SSTR3 and SSTR5; (Schmid et al., Neuroendocrinol. 2004;80:47-50). An especially preferred COMPOUND OF THE INVENTION is pasireotide or a pharmaceutically acceptable salt thereof.

It can be shown by established test models that the use of COMPOUND OF THE INVENTION results in an effective prevention and/or treatment of cluster headache.

In accordance with the particular findings of the invention, the present invention also provides a method of treating cluster headache in a subject in need thereof comprising administering to said subject a therapeutically effective amount of a COMPOUND OF THE INVENTION or a pharmaceutically acceptable salt thereof.

The present invention relates also to a pharmaceutical composition for treatment of cluster headache, comprising a therapeutically effective amount of a COMPOUND OF THE INVENTION or a pharmaceutically acceptable salt thereof, together with one or more pharmaceutically acceptable diluents or carriers.

The present invention relates also to a commercial package comprising a COMPOUND OF THE INVENTION together with instructions for use thereof in the treatment of cluster headache.

Pharmaceutical compositions for the treatment of cluster headache comprise an effective amount of the Somatostatin analog in free base form or in pharmaceutically acceptable salt form together with one or more pharmaceutically acceptable diluent or carrier. Such compositions may be formulated in conventional manner Somatostatin analogs may also be administered in sustained release form, e.g. in the form of implants, microcapsules, microspheres or nanospheres comprising e.g. a biodegradable polymer or copolymer, in the form of a liposomal formulation, or in the form of an autogel, e.g. a solid or semi-solid composition capable of forming a gel after interaction with patient's body fluids.

The COMPOUNDS OF THE INVENTION can, for example, be formulated as disclosed in WO05/046645 (especially pasireotide).

COMPOUNDS OF THE INVENTION or a pharmaceutically acceptable salt thereof may be administered by any conventional route, for example parenterally e.g. in form of injectable solutions or suspensions (including e.g. the sustained release form as indicated above), orally using a conventional absorption enhancer if necessary, in a nasal or a suppository form or topically, e.g. in the form of an ophthalmic liquid, gel, ointment or suspension preparation, e.g. a liposomal, microsphere or nanosphere formulation, e.g. for instillation or subconjunctival or intra- or peri-ocular injections.

The present pharmaceutical compositions are prepared in a manner known per se, and comprise approximately from 1 % to 100 %, preferentially from approximately 1 % to 40 %, especially from approximately 20 % to 30 %, active ingredient.

The structure of the active ingredients identified by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active ingredients and, based on these references, likewise enabled to manufacture and test the pharmaceutical indications and properties in standard test models, both *in vitro* and *in vivo.*

It will be understood that in the discussion of methods, references to the active ingredients are meant to also include the pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form salts with bases. Salts include acid addition salts with e.g. inorganic acids, polymeric acids or organic acids, for example with hydrochloric acid, acetic acid, lactic acid, aspartic acid, benzoic acid, succinic acid or pamoic acid. Acid addition salts may exist as mono- or divalent salts, e.g. depending whether 1 or 2 acid equivalents are added to the COMPOUND OF THE INVENTION in free base form. Preferred salts are tha lactate, aspartate, benzoate, succinate and pamoate including mono- and disalts, more preferably the aspartate di-salt and the pamoate monosalt, e.g. of pasireotide.

The active ingredient or a pharmaceutically acceptable salt thereof may also be used in form of a hydrate or include other solvents used for crystallization.

The person skilled in the pertinent art is fully enabled to select a relevant test model to prove the hereinbefore and hereinafter indicated therapeutic indications and beneficial effects.

The pharmacological activity of a COMPOUND OF THE INVENTION in cluster headache may, for example, also be demonstrated in clinical studies. Such clinical studies are preferably randomized, double-blind, clinical studies in patients suffering from cluster headache.

The effective dosage of the active ingredients employed may vary depending on the particular compound or pharmaceutical composition employed, the mode of administration, the severity of the condition being treated. Thus, the dosage regimen is selected in accordance with a variety of factors including the route of administration and the renal and hepatic function of the patient. A physician, clinician or veterinarian of ordinary skill can readily determine and prescribe the effective amount of the single active ingredients required to prevent, ameliorate or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients.

## Claims

1. Use of a Somatostatin (SRIF) analog which has a high binding affinity to human SSTR1,2,3,5, or a pharmaceutically acceptable salt thereof for the preparation of a pharmaceutical composition for the treatment of cluster headache.

2. Use according to claim 1 comprising as SRIF-analog pasireotide.

3. Method of treating cluster headache in a subject in need thereof, comprising administering to said subject a therapeutically effective amount of a Somatostatin (SRIF) analog which has a high binding affinity to human SSTR1,2,3,5, or a pharmaceutically acceptable salt thereof.

4. Method according to claim 3 comprising as SRIF-analog pasireotide.

5. A pharmaceutical composition for treatment of cluster headache, comprising a Somatostatin (SRIF) analog which has a high binding affinity to human SSTR1,2,3,5, or a pharmaceutically acceptable salt thereof, together with one or more pharmaceutically acceptable diluents or carriers therefor.

6. A pharmaceutical composition according to claim 5 comprising as SRIF-analog pasireotide.

7. A commercial package comprising a Somatostatin (SRIF) analog which has a high binding affinity to human SSTR1,2,3,5, or a pharmaceutically acceptable salt thereof, together with instructions for use thereof in the treatment of cluster headache.

8. A commercial package according to claim 7 comprising as SRIF-analog pasireotide.
